# EUROPEAN PATENT SPECIFICATION

(11) **EP 1 259 617 B1**
(45) Date of publication and mention of the grant of the patent: **14.04.2004**
(21) Application number: 01910943.8
(22) Date of filing: 16.02.2001
(51) Int. Cl.: C12N 15/40, C07K 14/175

(54) **BUNYAVIRIDAL REAPER PROTEINS AND USES THEREFOR**
REAPER PROTEINE DER BUNYAVIRIDAE SOWIE VERWENDUNGEN DIESER
PROTEINE VIRALE DE MORT CELLULAIRE ET UTILISATIONS ASSOCIEES

(30) Priority: 22.02.2000 US 184055 P
(43) Date of publication of application: 27.11.2002
(73) Proprietor: GLAXO GROUP LIMITED, Greenford, Middlesex UB6 ONN (GB)
(72) Inventor: SMITH, Gary, Keith, Glaxo Wellcome Inc., Research Triangle Park, NC 27709 (US)
(74) Representative: Giddings, Peter John, Dr.
(86) International application number: PCT/US2001/005275
(87) International publication number: WO 2001/062936

(56) References cited:
- WO-A-98/01563
- PEKOSZ ANDREW ET AL: "Induction of apoptosis by La Crosse virus infection and role of neuronal differentiation and human bcl-2 expression in its prevention." JOURNAL OF VIROLOGY, vol. 70, no. 8, 1996, pages 5329-5335, XP002178274 ISSN: 0022-538X
- KANG JU-IL ET AL: "Apoptosis is induced by hantaviruses in cultured cells." VIROLOGY, vol. 264, no. 1, 10 November 1999 (1999-11-10), pages 99-105, XP002178275 ISSN: 0042-6822
- GOYAL LAKSHMI ET AL: "Induction of apoptosis by Drosophila reaper, hid and grim through inhibition of IAP function." EMBO (EUROPEAN MOLECULAR BIOLOGY ORGANIZATION) JOURNAL., vol. 19, no. 4, 15 February 2000 (2000-02-15), pages 589-597, XP002178276 ISSN: 0261-4189
- CIPENS GUNARS ET AL: "Reaper gene RPR product has common elements of structure with gamma-invariant chain, p53, MMTV and M proteins." PROCEEDINGS OF THE LATVIAN ACADEMY OF SCIENCES SECTION B NATURAL EXACT, vol. 50, no. 4-5, 1996, pages 214-219, XP001025603 1996
- PLANCHON S M ET AL: "BCL-2 PROTECTS AGAINST BETA-LAPACHONE-MEDIATED CASPASE 3 ACTIVATIONAND APOPTOSIS IN HUMAN MYELOID LEUKEMIA (HL-60) CELLS" ONCOLOGY REPORTS, NATIONAL HELLENIC RESEARCH FOUNDATION, ATHENS, GR, vol. 6, no. 3, 1999, pages 485-492, XP000957471 ISSN: 1021-335X

## Description

### Field of the Invention

The present invention relates to a viral cell death gene and the protein expressed thereby, and in particular, but not exclusively, to nucleotide sequences, expression vectors, cell lines, antibodies, screening methods, compounds, methods of production and methods of treatment, related to them.

### Background of the Invention

Apoptosis is a form of programmed cell death, by which an organism eliminates extraneous or harmful cells. Apoptosis plays a role in normal development and homeostasis, as well as in diseases such as cancer and neurodegenerative diseases.

Apoptosis occurs via the activation of intrinsic cell-suicide programs; activation is regulated by many different signals, both intracellular and extracellular. Various viral and metazoan apoptosis inducer genes have been identified; inhibitors of apoptosis (IAP) proteins have also been identified that act to suppress apoptosis.

At least three apoptotic activator proteins have been identified in *Drosophila melanogaster*: reaper (rpr), head involution defective (hid) and grim. The N-terminal sequences of these proteins are highly conserved. Avdonin et al., *Proc*. *Natl*. *Acad. Sci. USA* 95:11703 (1998). Proteins that inhibit apoptosis (IAPs) have also been identified in Drosophila. Hay et al., *Cell* 83:1253 (1995); Wing et al., *Cell Death Differ.* 5:930 (1998).

The product of the reaper gene is required for programmed cell death in Drosophila. Cell death induced by the reaper protein has been shown to be blocked by the baculovirus p35 protein, a viral product that inactivates proteases. White et al., *Science* 271:805 (1996).

The Drosophila Grim protein is also an activator of apoptosis, independent of Reaper. Expression of Grim RNA coincides with the onset of programmed cell death during embryonic development, and ectopic induction of grim has been shown to trigger extensive apoptosis in transgenic animals and in cell culture. Similar to the Reaper protein, cell killing by grim can be blocked by coexpression of the viral p35 product. The grim gene product has been postulated to function in a parallel cell death signalling regime that activates a common set of downstream apoptotic effectors. Chen et al., Genes Dev. 10:1773-1782 (1996).

Viral infection of host cells, and replication therein, is often associated with inhibition of apoptosis to enable viral replication and the subsequent stimulation apoptosis of the host cells for viral particle release. Certain viral gene products have been shown to specifically inhibit or induce apoptosis. However, many viruses additionally encode proteins that inhibit apoptosis, prolonging the survival of infected cells and thereby aiding viral replication or viral persistence in the host.

### Summary of the Invention

In a first aspect, there is provided a method of screening a compound to determine whether the compound affects caspase activation induced by a viral reaper protein, comprising:
a) obtaining a vertebrate cell extract in which the addition of an isolated viral reaper protein induces detectable caspase activation;
b) adding an isolated viral reaper protein having at least 70% sequence similarity to SEQ ID NO:2 and capable of inducing caspase activation in a vertebrate cell, and a test compound to said vertebrate cell extract;
c) measuring caspase activation; and
d) comparing caspase activation that occurs in the presence of the test compound to that which would be expected in the absence of the test compound;
where a decrease in caspase activation indicates that said compound inhibits viral reaper-induced caspase activation, and an increase in caspase activation indicates that said test compound enhances viral reaper-induced caspase activation.

In a second aspect, a method of screening a compound to determine whether the compound affects apoptosis induced by a viral reaper protein, comprising:
a) obtaining a population of cells, the cells of a type in which the addition of an isolated viral reaper protein induces apoptosis;
b) administering an isolated viral reaper protein having at least 70% sequence similarity to SEQ ID NO:2 and capable of inducing caspase activation in a vertebrate cell, and a test compound to said cells;
c) measuring apoptosis that occurs in said cell population; and
d) comparing apoptosis that occurs in the presence of the test compound to that which would be expected in the absence of the test compound;
where a decrease in apoptosis indicates that said compound inhibits viral reaper-induced apoptosis, and an increase in apoptosis indicates that said test compound enhances viral reaper-induced apoptosis.

In a further aspect, there is provided a method of screening a compound to determine whether the compound affects apoptosis induced by a viral reaper protein, comprising:
a) obtaining a population of cells, the cells of a type in which the addition of an isolated viral reaper protein induces apoptosis;
b) transfecting said cells with a nucleotide molecule encoding a viral reaper protein having at least 70% sequence similarity to SEQ ID NO:2 and capable of inducing caspase activation in a vertebrate cell;
c) administering a test compound to said cells;
d) measuring apoptosis that occurs in said cell population; and
e) comparing apoptosis that occurs in the presence of the test compound to that which would be expected in the absence of the test compound;
where a decrease in apoptosis indicates that said compound inhibits viral reaper-induced apoptosis, and an increase in apoptosis indicates that said test compound enhances viral reaper-induced apoptosis.

In a further aspect, there is provided a method of screening a compound for viral reaper modulating activity, comprising:
(a) obtaining a population of cells transfected with an isolated nucleotide molecule encoding a viral reaper protein, where said viral reaper protein has at least 70% sequence similarity to SEQ ID NO:2, and is capable of inducing caspase activation in a vertebrate cell;
(b) administering to a test population of said cells a test compound; and
(c) comparing apoptosis occurring in said test population of cells to that which would be expected in a population of cells that did not receive said test compound;
where a reduction in apoptosis indicates that said test compound inhibits viral reaper-induced apoptosis, and an increase in apoptosis indicates said test compound enhances viral-reaper induced apoptosis.

In a further aspect, there is provided a method of inducing apoptosis in a vertebrate cell in vitro by administering an isolated viral reaper protein to said cell, in an amount sufficient to enhance apoptosis over that which would be seen in the absence of viral reaper protein, where said viral reaper protein has at least 70% sequence similarity to SEQ ID NO:2, and is capable of inducing caspase activation in a vertebrate cell.

In a further aspect, there is provided a method of inducing apoptosis in a vertebrate cell in vitro by transfecting said cell with an isolated nucleic acid molecule encoding a viral reaper protein, in an amount sufficient to enhance apoptosis over that which would be seen in the absence of transfection, where said encoded viral reaper protein has at least 70% sequence similarity to SEQ ID NO:2 and is capable of inducing caspase activation in a vertebrate cell.

### Brief Description of the Figures

The present invention will be further described by the way of example and with reference to the following figures:

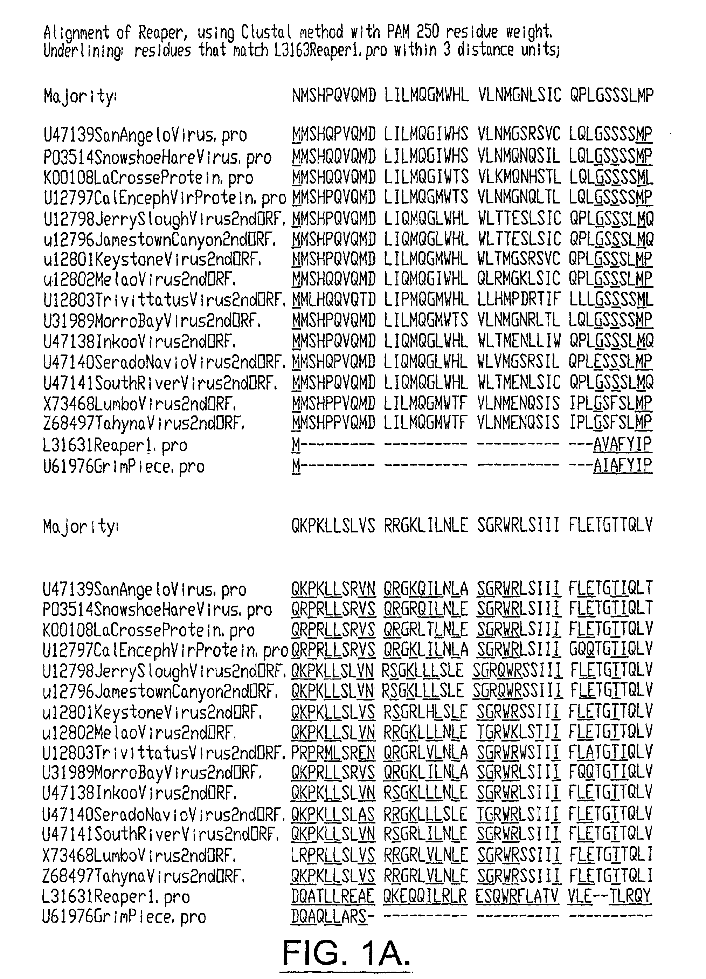

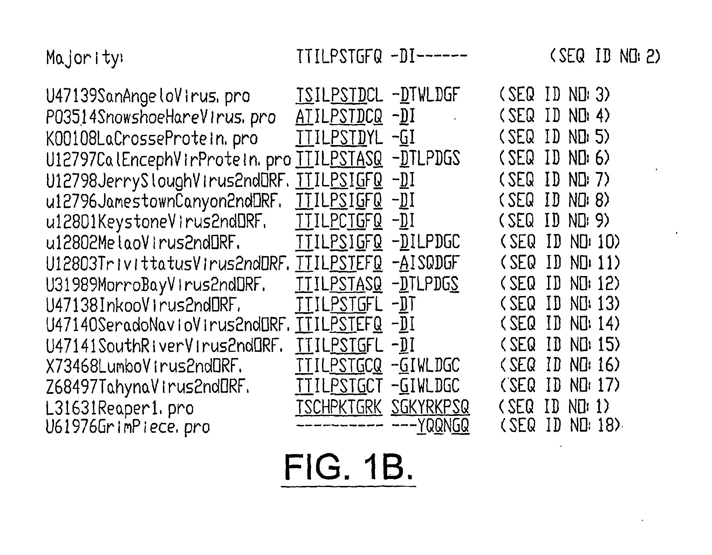

**Figure 1** shows the amino acid sequences of the non structural (NSs) viral proteins from fifteen different virus, aligned with the amino acid sequence of the complete Reaper protein (SEQ ID NO:1) and a portion of the Grim protein from the *Drosophila melanogaster* (SEQ ID NO:18). A majority sequence for the viral reaper protein is provided as SEQ ID NO:2.

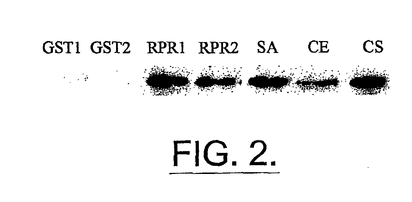

**Figure 2** shows Western blot results indicating binding of various reaper proteins to Xenopus scythe protein (Example 4). RPR1 and RPR2 indicate preparations of Drosophila Reaper, SA indicates San Angelo virus reaper, and CE indicates California Encephalitis virus reaper; GST1 and GST2 are preparations of glutathione-S-transferase used as controls.

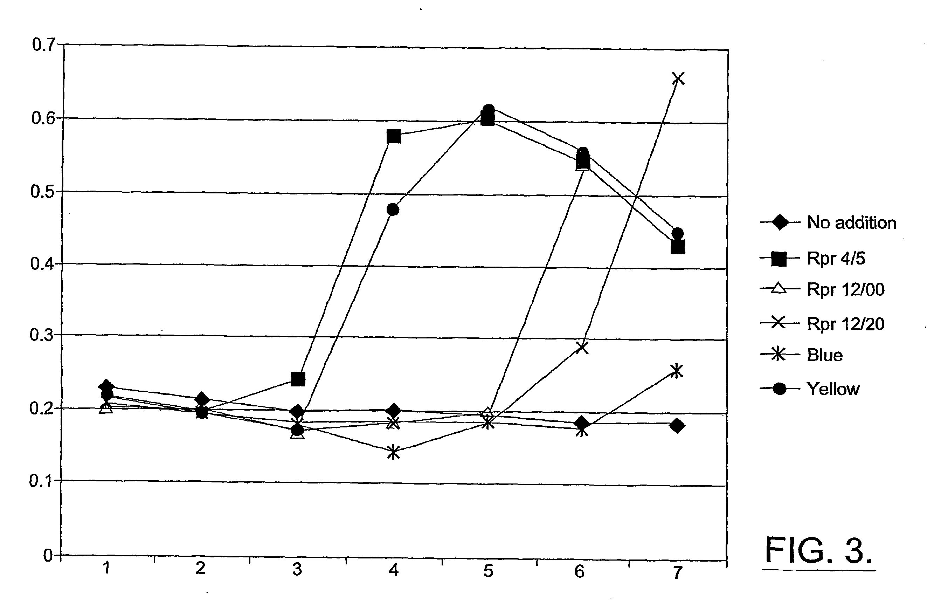

**Figure 3** graphs the results of a colorimetric assay to detect caspase activation in Xenopus cell-free extracts with added Drosophila reaper (Rpr 4/5, Rpr 12/00, Rpr 12/20), San Angelo virus reaper (SA), and California Encephalitis virus reaper (CE). Glutathione-S-transferase (GST) was used as a control. The Y-axis shows absorbance at 405 nm; the X-axis = time in hours.

### Detailed Description of the Invention

Viral proteins with sequence similarity to the *Drosophila melanogaster* Reaper protein have been identified, and shown to be capable of activating caspases in vertebrate cell-based assay. Cellular death by apoptosis is, with only some exceptions, executed by the family of proteases known as caspases. The caspases are synthesized as inactive zymogens and activated during apopototic pathways. (Chinnaiyan and Dixit, *Curr. Biol*. 6:555 (1996); Muzio et al, *J*. *Biol*. *Chem*. 273:2926 (1998); Yang et al., *Mol*. *Cell*. 1:319 (1998); Zou et al., *Cell* 90:405 (1997)). Caspase activation (and thus ultimately apoptosis) can be blocked by the IAP (inhibitor of apoptosis) proteins and anti-apoptotic members of the Bcl-2 family (Adams and Cory *Science* 281:1322 (1998); Deveraux and Reed, *Genes Dev.* 13:239 (1999)).

The present viral genes and their expression products provide useful screening assays for the identification and development of novel pharmaceutical agents, including agonists and antagonists of the viral reaper proteins, and compounds able to enhance or inhibit caspase activation and/or cellular apoptosis; such compunds may be used in the treatment and/or prophylaxis of disorders such as cancer and viral infections. In particular, the present methods are useful in identifying compounds that enhance or inhibit apoptosis due to caspase activation.

Accordingly, it is an object of the present invention to provide methods of using isolated viral reaper proteins. Other objects of the present invention will become apparent from the following detailed description thereof.

The present inventors identified viral proteins with sequence similarity to the *D. melanogaster* Reaper protein. More particularly, the present inventors determined that the non-structural protein (NSs) of viruses of the genus Bunyavirus exhibited sequence similarity to the Drosophila Reaper protein.

Viruses of the genus Bunyavirus (family Bunyaviridae) are single stranded RNA negative-strand viruses that infect vertebrates. There are 18 antigenic groups of the genus Bunyavirus (at least 161 viruses) and several additional ungrouped viruses.

The California encephalitis virus, LaCrosse virus, San Angelo virus, Snowshoe Hare virus, Jerry Slough virus, Jamestown Canyon virus, Keystone Virus, Melao virus, Trivittatus virus, Morro Bay virus, Inkoo virus, Serra do Navio virus, South River virus, Lumbo virus, and Tahyna virus are each members of the genus Bunyavirus. The genome consists of a large (L) RNA, a medium (M) RNA, and a small (S) RNA. The nonstructural protein (NSs protein) of these viruses is encoded by the small (S) RNA, and is induced in virus-infected cells (Fuller and Bishop (1982) J. Virol. 41, 643-648). The NSs protein of each of these viruses was found by the present inventors to have sequence similarity to the Drosophila Reaper protein (**Figure 1**).

A nucleotide sequence encoding *D. melanogaster* Reaper protein is given at GenBank Accession No. L31631; the protein sequence is provided at Acc. No. AAA18983. The amino acid sequence of the Drosophila cell death protein GRIM is provided at GenBank Acc. No. AAC47727.

The nucleotide sequence encoding San Angelo virus (Prototype VR723) nucleocapsid and non-structural proteins is provided at GenBank Accession U47139. The amino acid sequence of San Angelo virus NSs protein is provided at Acc No. AAC5335.

The amino acid sequence of a NSs of the Snowshoe Hare virus is provided at Accession No. P03514; the nucleotide sequence of a small (S) viral RNA species of snowshoe hare virus is provided at Acc. No. J02390. See also Bishop et al., *Nucleic Acids Res.* 10 (12), 3703-3713 (1982).

The nucleotide sequence of a La Crosse virus small RNA segment is provided at GenBank Accession No. K00108. The amino acid sequence for a La Crosse Virus non structural protein can be found at Accession No. AAA42780.1

The nucleotide sequence for a California Encephalitis virus small RNA segment, including the nucleocapsid and NSs protein genes, is provided at GenBank Accession No U12797; the amino acid sequence of the viral NSs protein can be found at Acc. No. AAC54056.1. See also Bowen,M.D., et al. *J*. *Gen. Virol*. 76 : 559-572 (1995).

The nucleotide sequence encoding Jerry Slough Virus NSs protein is provided at GenBank Accession No. U12798; the amino acid sequence of the viral NSs protein can be found at Acc. No. AAC54048.

The nucleotide sequence encoding Jamestown Canyon Virus NSs protein is provided at GenBank Accession No. U12796; the amino acid sequence of the viral NSs protein can be found at Acc. No. AAC54044.

The nucleotide sequence encoding Keystone Virus NSs protein is provided at GenBank Accession No. U12801; the amino acid sequence of the viral NSs protein can be found at Acc. No. 54054.

The nucleotide sequence encoding Melao Virus NSs protein is provided at GenBank Accession No. U12802; the amino acid sequence of the viral NSs protein can be found at Acc. No. AAB60560.

The nucleotide sequence encoding Trivittatus Virus NSs protein is provided at GenBank Accession No. U12803; the amino acid sequence of the viral NSs protein can be found at Acc. No. AAB60562.

The nucleotide sequence encoding Morro Bay Virus NSs protein is provided at GenBank Accession No. U31989; the amino acid sequence of the viral NSs protein can be found at Acc. No. AAC55125.

The nucleotide sequence encoding Inkoo Virus NSs protein is provided at GenBank Accession No. U47138; the amino acid sequence of the viral NSs protein can be found at Acc. No. AAC55333.

The nucleotide sequence encoding Serra do Navio Virus NSs protein is provided at GenBank Accession No. U47140; the amino acid sequence of the viral NSs protein can be found at Acc. No. AAC55337.

The nucleotide sequence encoding South River Virus NSs protein is provided at GenBank Accession No. U47141; the amino acid sequence of the viral NSs protein can be found at Acc. No. AAC55339.

The nucleotide sequence encoding Lumbo Virus NSs protein is provided at GenBank Accession No. X73468; the amino acid sequence of the viral NSs protein can be found at Acc. No. CAA51852.

The nucleotide sequence encoding Tahyna Virus NSs protein is provided at GenBank Accession No. Z68497; the amino acid sequence of the viral NSs protein can be found at Acc. No. Z68497.

The present viral Reaper proteins act as apoptotic modulating proteins, affecting apoptosis in host cells. Discovery of these viral proteins provides methods to screen compounds for the ability to block or enhance the viral reaper function, and the use of compounds identified thereby to modulate apoptosis. Apoptosis may also be modulated by the administration of viral reaper proteins (or a functional variant or fragment thereof) to a cell. Administration may utilize isolated nucleic acid molecules encoding the viral reaper proteins, vectors containing such molecules, and host cells transfected with the same. The host cells may be any cell suitable for cultivation of the virus, as is known in the art. More preferred are vertebrate cells, including mammalian cells.

The present proteins (and functional variants and fragments thereof) and nucleotides expressing the same, are useful in several settings. Where a virus is being cultivated, either in cell culture or *in vivo,* the presently identified viral reaper proteins (and/or the compounds discovered in the screening assays described above) may be used to modulate apoptosis of infected host cells, by administering the protein or compounds to the cells. Inhibiting apoptosis of the host cell is useful where further replication of the virus within the host cell can be attained and is desirable. Inducing apoptosis of the host cell is useful where infection of additional cells is desirable, in harvesting virus, or where it is necessary and desirable to terminate survival of the host cell in order to halt further viral replication/propagation.

Additionally, the present proteins and/or compounds discovered using the present screening assays, may be used in the treatment of certain conditions involving aberrant apoptosis of a subject's cells. Viral reaper proteins and/or such compounds may be administered to a preselected population of cells in a subject, to modulate apoptosis of those cells. Such a method is useful in conditions where the normal apoptotic mechanism of the subject's cells is altered, e.g., in cancer and neoplastic growths. Such a method is also useful in treating viral infections.

The present invention also provides a method to screen compounds for the ability to inhibit or enhance the function of the viral reaper proteins (e.g., to act as antagonists or agonists of the viral reaper protein function). Such compounds will be useful in the above-described methods. Such compounds may be administered alone, to act upon endogenous viral reaper proteins produced within an infected host cell; or they may be administered to a cell in conjunction with exogenous viral reaper protein where needed (e.g., where the compound enhances the effects of the exogenous viral reaper protein).

As used herein, "sequence similarity" of proteins refers to the similarity of the amino acid sequence between two proteins. Various computer programs are commercially available that determine sequence similarity, and are known to those skilled in the art. The degree of sequence similarity takes into consideration both amino acid residues that are identical, as well as those that are conservative amino acid substitutions (as is known in the art).

"Sequence similarity" as used in the present specification and claims refers to DNA sequences, ( or RNA sequences; or amino acid sequences) that have only slight and non-consequential sequence variations between them. In this regard, 'slight and non-consequential sequence variations' mean that the sequences will be functionally equivalent. Functionally equivalent sequences will function in substantially the same manner to produce substantially the same results.

As used herein, two amino acid sequence that have substantial "sequence similarity" are those having at least about 50% or 55% sequence similarity, and preferably at least about 60%, 65%, 70%, 75%, 80%, 85%, or even about 90% or 95% similarity. Changes in the amino acid sequence of peptides can be guided by known similarities among amino acids and other molecules or substituents in physical features such as charge density, hydrophobicity, hydrophilicity, size and configuration, etc. For example, the amino acid Thr may be replaced by Ser and vice versa, and Leu may be replaced by Ile and vice versa.

The peptides of the present invention include not only natural amino acid sequences, but also peptides which are analogs, chemical derivatives, or salts thereof. The term "analog" or "conservative variation" refers to any polypeptide having a substantially identical amino acid sequence to the peptides identified herein, and in which one or more amino acids have been substituted with chemically similar amino acids. For example, a polar amino acid such as glycine or serine may be substituted for another polar amino acid; a basic amino acid may be substituted for another basic amino acid, or an acidic amino acid may be substituted for another acidic amino acid; or a non-polar amino acid may be substituted for another non-polar amino acid. There term "analog" or "conservative variation" as used herein also refers to a peptide which has had one or more amino acids deleted or added to a polypeptide of the present invention, but which retains a substantial sequence similarity (at least about 85% sequence similarity, and preferably at least 90%, 95%, 98% or even 99% sequence similarity), where the peptide retains the viral reaper protein function or generates an antagonistic viral reaper function.

Given the amino acid sequence of a particular viral protein, a nucleotide sequence encoding the protein can be readily determined, and a nucleotide molecule encoding the protein can be prepared.

The present invention also encompasses the use of nucleotide molecules that encode viral reaper proteins. Examples of such nucleotide sequences are below. Due to the degeneracy of the genetic code, one skilled in the art will be able to readily devise alternative nucleotide sequences that encode a given protein, where the amino acid sequence of the protein is known.
nucleotide sequence encoding Drosophila melanogaster Reaper Protein L31631
nucleotide sequence encoding San Angleo Virus NSs protein U47139
nucleotide sequence encoding Snowshoe Hare Virus UP03514
nucleotide sequence encoding LaCrosse Virus NSs protein K00108
nucleotide sequence encoding California encephalitis NSs protein U12797
nucleotide sequence encoding Jerry slough virus NSs protein U12798
Nucleotide sequence encoding Jamestown Canyon virus NSs protein U12796
Nucleotide sequence encoding Keystone Virus NSs protein U12801
Nucleotide sequence encoding Melao Virus NSs protein U12802
Nucleotide sequence encoding Trivittatus Virus NSs protein U12803
Nucleotide sequence encoding Morro Bay Virus NSs protein U31989
Nucleotide sequence encoding Inkoo Virus NSs protein U47138
Nucleotide sequence encoding Serra do Navio Virus NSs protein U47140
Nucleotide sequence encoding South River Virus NSs protein U47141
Nucleotide sequence encoding Lumbo Virus NSs protein X73468
Nucleotide sequence encoding Tahyna Virus NSs protein Z68497

Nucleotide sequences that have substantial sequence similarity to the nucleotide sequences disclosed herein, and that encode functional viral reaper proteins, are suitable for use in the methods of the present invention.

Thus, use of nucleotides that hybridize to nucleotide molecules encoding the viral reaper proteins of SEQ ID NOs:2-17 are also an aspect of this invention. Conditions which will permit other nucleotide molecules encoding viral reaper proteins to hybridize to the nucleotide sequences disclosed herein can be determined in accordance with known techniques. For example, hybridization of such sequences may be carried out under conditions of reduced stringency, medium stringency, or even high stringency conditions (e.g., conditions represented by a wash stringency of 35-40% formamide with 5x Denhardt's solution, 0.5% SDS and 1x SSPE at 37°C; conditions represented by a wash stringency of 40-45% formamide with 5x Denhardt's solution, 0.5% SDS, and 1x SSPE at 42°C; and conditions represented by a wash stringency of 50% formamide with 5x Denhardt's solution, 0.5% SDS and 1x SSPE at 42 °C, respectively, to a nucleotide molecule encoding a viral reaper protein as disclosed herein in a standard hybridization assay. See J. Sambrook et al., Molecular Cloning: A Laboratory Manual (2d ed. 1989)). In general, sequences encoding functional viral reaper proteins that hybridize to the nucleotide sequences disclosed herein will have at least 75% sequence similarity, and even 95% sequence similarity or more with the nucleotide sequences encoding viral reaper proteins disclosed herein.

Throughout the present specification and the accompanying claims the words "comprise" and "include" and variations such as "comprises", "comprising", "includes" and "including" are to be interpreted inclusively. That is, these words are intended to convey the possible inclusion of other elements or integers not specifically recited, where the context allows.

As referred to above, the present invention relates to methods of using isolated viral reaper proteins or isolated nucleotide molecules encoding viral reaper proteins. In the context of this invention the term "isolated" is intended to convey that the protein is not in its native state, insofar as it has been purified at least to some extent or has been synthetically produced, for example by recombinant methods. The term "isolated" therefore includes the possibility of the protein being in combination with other biological or non-biological material, such as cells, suspensions of cells or cell fragments, proteins, peptides, expression vectors, organic or inorganic solvents, or other materials where appropriate, but excludes the situation where the protein or nucleic acid molecule is in a state as found in nature.

As used herein, the term "viral reaper protein" comprises proteins having an amino acid sequence identical to that of a protein naturally expressed by a virus, and having the ability to induce caspase activation in a vertebrate cell. One method of assaying a protein for the ability to induce caspase activation in a vertebrate cell (Xenopus oocytes) is provided in the Examples section herein.

Routine methods, as further explained in the subsequent experimental section, can be employed to purify and/or synthesize the proteins according to the invention. Such methods are well understood by persons skilled in the art, and include techniques such as those disclosed in Sambrook J., Fritsch E.F. and Maniatis T., Molecular Cloning: a Laboratory Manual; 2^{nd} Edition; CSH Laboratory Press (1989), the disclosure of which is included herein in its entirety by way of reference.

The term "variant" as used herein refers to peptides or proteins which retain the same essential characteristics (functional and structural) of the viral Reaper proteins for which sequence information is provided herein; such variants are intended to be included within the scope of the invention. For example, other peptides or proteins with greater than about 65%, preferably at least 75% and particularly preferably at least 80%, 90% or 95% sequence similarity with the sequences provided, are considered as variants of the proteins. Such variants may include the deletion, modification or addition of single amino acids or groups of amino acids within the protein sequence, as long as the peptide maintains the basic biological functionality of a viral Reaper protein. This biological functionality can be assessed by one skilled in the art using methods that are known in the art.

The term "protein" as used herein is also intended to include within its meaning shorter peptide or polypeptide sequences as well as complete proteins. For example therefore a peptide of only perhaps 15 amino acids in length is considered to fall within the scope of the invention as long as it demonstrates the basic biological functionality of a viral Reaper protein as described herein. In particular, but not exclusively, this aspect of the invention encompasses the situation when the protein is a fragment of the complete protein sequence and may represent a ligand binding region.

The invention also includes methods of using isolated nucleotide sequences that encode viral Reaper proteins or variants thereof, as well as isolated nucleotide sequences which are complementary thereto. The nucleotide sequence may be RNA or DNA including genomic DNA, synthetic DNA or cDNA. Preferably the nucleotide sequence is a DNA sequence and most preferably, a cDNA sequence. Nucleotide sequence information is provided herein for certain viral reapers. Such nucleotides can be isolated from virally infected cells or synthesised according to methods well known in the art, as described by way of example in Sambrook J, Fritsch E.F. and Maniatis T; Molecular Cloning: a Laboratory Manual; 2^{nd} Edition; CSH Laboratory Press (1989), the disclosure of which is included herein in its entirety by reference. The nucleotide molecules according to the invention have utility in production of the proteins according to the invention, which may take place *in vitro, in vivo* or *ex vivo*. The nucleotides may be involved in recombinant protein synthesis or indeed as therapeutic agents in their own right, utilised in gene therapy techniques. Nucleotides complementary to those encoding viral Reaper proteins of the present invention, or antisense sequences, may also be used in therapy, such as in strategies for down regulation of expression of the proteins of the invention.

The present invention also includes methods of using expression vectors that comprise nucleotide sequences encoding viral Reaper proteins or variants thereof. Such expression vectors are routinely constructed in the art of molecular biology and may for example involve the use of plasmid DNA and appropriate initiators, promoters, enhancers and other elements, such as for example polyadenylation signals which may be necessary, and which are positioned in the correct orientation, in order to allow for protein expression. Suitable vectors would be apparent to persons skilled in the art. By way of further example in this regard we refer to Sambrook J, Fritsch E.F. and Maniatis T; Molecular Cloning: a Laboratory Manual; 2^{nd} Edition; CSH Laboratory Press (1989), the disclosure of which is included herein in its entirety.

The invention also includes methods of using cell lines that have been modified to express viral reaper proteins. Such cell lines include transient, or preferably stable higher eukaryotic cell lines, such as vertebrate cells, mammalian cells or insect cells; lower eukaryotic cells, such as yeast; or prokaryotic cells such as bacterial cells. Preferred are bacterial, insect and vertebrate cells. As used herein, cells that have been "modified" to express viral reaper proteins are those that contain isolated nucleic acid molecules coding for a viral reaper protein, and excludes cells infected with a virus expressing viral reaper protein from a non-isolated nucleic acid (e.g., a naturally occurring virus).

A further aspect of the present invention is the use of the proteins according to the invention in screening methods designed to identify those compounds that act as ligands for viral Reaper proteins, and/or that modulate viral Reaper protein activity. In general terms, such screening methods will involve contacting the protein concerned, or cell modified to express the protein concerned, with a test compound and then detecting any enhancement or inhibition of protein activity that results (compared to the activity that would occur in the absence of the test compound). The present invention also includes within its scope those compounds that are identified as possessing useful viral Reaper protein modulation activity, by the screening methods referred to above, and use of such compounds. The screening methods comprehended by the invention are generally well known to persons skilled in the art.

Methods of screening compounds comprise contacting a viral reaper protein with a test compound, or administering a test compound to a cell that contains or expresses a viral reaper protein. By contacting it is meant that the test compound and viral reaper protein are in such proximity that they are able to biologically interact. Administration of a compound to a cell refers to the placement of the compound within the cell interior, either by direct administration or by cellular uptake.

A particular method of screening a compound to determine whether the compound enhances or inhibits caspase activation utilizes vertebrate cell extracts (cell-free preparations) which are known to exhibit detectable caspase activation when functional viral reaper is added to the cell extract. As used herein, an increase in (or enhancement of) caspase activation includes an increase in total caspase activity, a faster rate of caspase activity, and/or a decreased time until caspase activity is detected, as well as other measures that will be apparent to those skilled in the art. As used herein, a decrease in (or inhibition of ) caspase activation includes a decrease in total caspase activity, a slowed rate of caspase activity, and/or an increase in the time until caspase activity is first detected; as well as other measures that will be apparent to those skilled in the art. Other indicators of activation of the apoptotic pathway are known in the art, e.g., mitochondrial cytochrome c release, fragmentation of nuclei added to a cell extract preparation. The change in caspase activation (or other indicators of apoptosis) is compared between preparations that contain the test compound and control preparations that do not; however, such comparisons need not be a side-by-side comparison, where a control has previously been tested and has provided data for comparison.

As used herein, a compound with "viral reaper modulating activity" is one that is capable of enhancing or inhibiting viral reaper induced apoptosis or caspase activation. As used herein, apoptosis induced by a viral reaper protein is that caused by activation of the apoptotic pathway by the viral reaper.

The present invention will now be further described by way of example.

### EXAMPLES

### Example 1

### Identification of Viral Reaper Proteins

Using Advanced Blast, Psi Blast, Edit Seq and Meg Align programs, 15 nonstructural viral proteins (or the *in silico* translated viral DNA for these proteins) were unexpectedly found to have sequence similarity to the *Drosophila melanogaster* Reaper protein.
The 15 viral proteins *(or in silico* translated DNAs) investigated were the non-structural proteins (NSs) of San Angelo virus (NSs) (SEQ ID NO:3); Snow Shoe Hare virus (SEQ ID NO:4); La Crosse virus (SEQ ID NO:5), California encephalitis virus (SEQ ID NO:6), Jerry Slough virus (SEQ ID NO:7), Jamestown Canyon virus (SEQ ID NO:8), Keystone virus (SEQ ID NO:9), Melao virus (SEQ ID NO:10), Trivittatus virus (SEQ ID NO:11), Morro Bay virus (SEQ ID NO:12), Inkoo virus (SEQ ID NO:13), Serra do Navio virus (SEQ ID NO:14), South River virus (SEQ ID NO:15), Lumbo virus (SEQ ID NO:16), and Tahyna virus (SEQ ID NO:17). The amino acid sequences of these proteins were compared to that of the *D. melanogaster* Reaper protein (SEQ ID NO:1) and a portion of the *D. melanogaster* Grim protein (SEQ ID NO:18) Results are shown in **Figure 1**.

It was found that 59 amino acids of the 65 amino acid drosophila reaper protein aligned with 61 amino acids of the ∼ 92 amino acid viral reaper protein (or *in silico* translated DNA) with 58 % similarity and 29 % identity. These points of alignment therefore provide for a consensus reaper sequence cross-species.

A majority sequence was prepared (SEQ ID NO:2).

### Example 2:

### Function of viral reaper proteins.

Viral reaper activity is detected utilizing extracts prepared from Xenopus eggs as described in Thress, et al, 1998, *The EMBO Journal* 17:6135-6143. Briefly, an egg extract prepared as described, is treated with 10 to 1000 nanograms of viral reaper protein or a glutathione-S transferase (GST) fusion protein with viral reaper. Control extracts (without viral reaper) are also prepared. After incubation of the mixture, caspase enzyme activity is measured with the substrate DEVD-pNA by following the increase in absorbance at 405 nanometers. Proteins such as reaper that are apoptotic activators cause an acceleration of the production of caspase activity (compared to that seen in controls).
The presence of apoptotic inhibitors slows or reduces caspase activation in extracts where reaper is incubated with the extract (compared to extracts incubated with reaper that do not contain the apoptotic inhibitor).
Ectopic expression of Drosophila Reaper has also been shown to induce caspase activation and apoptosis in mammalian cells such as human MCF7 breast carcinoma cells (McCarthy & Dixit, *J*. *Biol*. *Chem.* 273:24009 (1998)).

### Example 3

### Screening for compounds that exhibit Viral Reaper modulating activity

A cell culture of cells susceptible to viral Reaper-induced effects is established. The viral Reaper protein is administered to the cells (e.g., via transfection with a suitable plasmid and optionally a reporter protein such as B-galactosidase; or by direct administration of the viral reaper protein to the cell). A test compound is also administered to a test population of the cells. Administration of the test compound may occur prior to, concurrently with, or after administration of the viral Reaper protein. The effect of administration of the test compound on apoptosis of cells is compared to a control population of cells that did not receive the test compound. Significant differences in apoptosis (and/or caspase activation or other indicators of apoptosis) between the test and control cells indicates that the test compound modulates viral reaper protein activity.
Alternatively, extracts prepared from Xenopus eggs are used as described herein to test compounds for viral reaper-modulating effects. The test compound is included in an assay as described herein, and caspase activity in cells receiving the test compound is compared to control cells that do not receive the test compound. Significant differences in caspase activity production between the test and control extracts indicates that the test compound modulates viral reaper protein activity.

### Example 4

### Binding of Drosophila and Viral Reaper to Scythe

Glutathione S-transferase, Drosophila reaper, and two viral reaper proteins were examined to detect the ability to bind to scythe protein. It has previously been shown that drosophila reaper binds to Xenopus scythe protein to induce apoptosis in Xenopus oocytes. Scythe has been indicated as an apoptotic regulator that is an essential component in reaper-induced apoptosis; immunodepletion of scythe from Xenopus egg extracts prevents reaper-induced apoptosis without affecting apoptosis triggered by activated caspase. (Thress et al., *EMBO J* 17:6135-43 (1998); Evans et *al,EMBO J* 16:7372-81 (1997); Thress et al., *EMBO J* 18:5486 (1999)).

Glutathione S-transferase (GST), a Drosophila Reaper-GST construct (RPR), San Angelo virus reaper - GST construct (SA), and California Encephalitis virus reaper - GST construct (CE) were produced in BL21 bacterial strains (using standard techniques as are known in the art) and purified using glutathione sepharose beads. Two preparations of GST prepared by two different individuals were utilized as controls (GST1 and GST2); two preparations of Drosophila reaper prepared by two individuals were also used (RPR1 and RPR2). The Xenopus crude egg extract was prepared as described in Evans et al., *EMBO J,* 16:7372 (1997) and Thress et al., *EMBO J*., 17:6135 (1998).

The beads were incubated in Xenopus crude egg extract for an hour. The beads were then washed several times in egg lysis buffer (ELB; 250 mM sucrose, 2.5 mM MgCl2, 1.0 mM dithiothreitol (DTT), 50 mM KCl, 10 mM HEPES), pH 7.4, boiled, and run in an SDS-PAGE gel (using techniques as are well-known in the art) and subjected to Western analysis (using techniques that are well known in the art) to detect binding of scythe to the beads. An aliquot of Xenopus crude egg extract (CS) without any reaper or GST was also run on SDS-PAGE gel as a positive control.

As shown in **Figure 2**, no binding was seen with either of the two GST preparations. Drosophila reapers (RPR1 and RPR2) bound scythe; the Xenopus crude egg extract (CS), which contains scythe, was included to show the position of scythe. Additionally, both of the viral reapers (SA and CE) showed scythe binding. The reduced intensity of the Western blot corresponding to viral CE reaper may indicate reduced binding to scythe (compared to the other reaper proteins tested) in this assay, as total reaper or viral reaper protein was maintained constant.

### Example 5

### Activation of Caspase

Caspase activation was measured using three separate preparations of Drosophila Reaper-GST, San Angelo virus reaper-GST (SA), and California Encephalitis virus reaper -GST(CE) (prepared and purified as described above). GST was used as a control.

Cell-free extracts were prepared from Xenopus oocytes as described in Thress et al., *EMBO J*., 17:6135 (1998), at page 6141. While it is known that these cell-free extracts will spontaneously release mitochondrial cytochrome c and activate endogenous caspases after prolonged incubation at room temperature, it has further been shown that the addition of drosophila Reaper measurably accelerates this process, triggering mitochondrial cytochrome c release, caspase activation, and fragmentation of added nuclei. (Newmeyer et al., *Cell* 79:353 (1994); Evans et al., *EMBO J*. 16:7372 (1997)).

Briefly to assess the ability of a reaper or viral reaper protein to induce caspase release, and thus by inference induce apoptosis, 10 ul of GST-reaper or GST-viral reaper protein (released from GST beads with glutathione under standard conditions) at ∼0.5 to 1 mg/ml was mixed with 100 ul *Xenopus* oocyte extract. This mixture was incubated for up to 7 hr and assayed for caspase activation a specific times. To measure the caspase activity in the incubation mixtures, 3µl of each incubation sample were mixed and incubated with 90µl of assay buffer (50 mM HEPES pH 7.5, 100 mM NaCl, 0.1% CHAPS, 10 mM DTT, 1mM EDTA, 10% glycerol) and the colorimetric substrate Ac-DEVD-pNA (final concentration 200 µM) [BioMol Caspase-3 assay system; BioMol Research Laboratories Inc., Plymouth Meeting, Pennsylvania] at 37°C. At various time points, absorbance was measured at 405 nm; the measure of absorbance is directly proportional to caspase activation.

As shown by **Figure 3** (Y-axis = absorbance at 405 nm; X-axis = time (in hours ), the GST control did not result in activation of caspase, while each of the three Drosophila reaper preparations activated caspase, although the time at which activation was first detected varied among the reaper preparations. Both of the viral reaper preparations resulted in caspase activation. The SA virus reaper activated caspase over a time course similar to that of the Reaper 4/5 preparation in this assay. The CE virus reaper resulted in initial caspase activation at the 6-7 hour timepoint, however, the experiment did not extend beyond this time point.

## Claims

1. A method of screening a compound to determine whether the compound affects caspase activation induced by a viral reaper protein, comprising:
a) obtaining a vertebrate cell extract in which the addition of an isolated viral reaper protein induces detectable caspase activation;
b) adding an isolated viral reaper protein having at least 70% sequence similarity to SEQ ID NO:2 and capable of inducing caspase activation in a vertebrate cell, and a test compound to said vertebrate cell extract;
c) measuring caspase activation; and
d) comparing caspase activation that occurs in the presence of the test compound to that which would be expected in the absence of the test compound;
where a decrease in caspase activation indicates that said compound inhibits viral reaper-induced caspase activation, and an increase in caspase activation indicates that said test compound enhances viral reaper-induced caspase activation.

2. A method according to claim 1 where said isolated viral reaper protein comprises an amino acid sequence selected from SEQ ID NOs: 2-17.

3. A method according to claim 1 where said isolated viral reaper protein is a non-structural protein (NSs) encoded by the viral small RNA from a virus of the Family Bunyaviridae.

4. A method according to claim 1 where said vertebrate cell extract is obtained from Xenopus oocytes.

5. A method of screening a compound to determine whether the compound affects apoptosis induced by a viral reaper protein, comprising:
a) obtaining a population of cells, the cells of a type in which the addition of an isolated viral reaper protein induces apoptosis;
b) administering an isolated viral reaper protein having at least 70% sequence similarity to SEQ ID NO:2 and capable of inducing caspase activation in a vertebrate cell, and a test compound to said cells;
c) measuring apoptosis that occurs in said cell population; and
d) comparing apoptosis that occurs in the presence of the test compound to that which would be expected in the absence of the test compound;
where a decrease in apoptosis indicates that said compound inhibits viral reaper-induced apoptosis, and an increase in apoptosis indicates that said test compound enhances viral reaper-induced apoptosis.

6. A method according to claim 5 wherein said population of cells consists of vertebrate cells.

7. A method according to claim 5 where said isolated viral reaper protein comprises an amino acid sequence selected from SEQ ID NOs: 2-17.

8. A method according to claim 5 where said isolated viral reaper protein is a non-structural protein (NSs) encoded by the viral small RNA from a virus of the Family Bunyaviridae.

9. A method of screening a compound to determine whether the compound affects apoptosis induced by a viral reaper protein, comprising:
a) obtaining a population of cells, the cells of a type in which the addition of an isolated viral reaper protein induces apoptosis;
b) transfecting said cells with a nucleotide molecule encoding a viral reaper protein having at least 70% sequence similarity to SEQ ID NO:2 and capable of inducing caspase activation in a vertebrate cell;
c) administering a test compound to said cells;
d) measuring apoptosis that occurs in said cell population; and
e) comparing apoptosis that occurs in the presence of the test compound to that which would be be expected in the absence of the test compound;
where a decrease in apoptosis indicates that said compound inhibits viral reaper-induced apoptosis, and an increase in apoptosis indicates that said test compound enhances viral reaper-induced apoptosis.

10. A method according to claim 5 where apoptosis is measured by a criterion selected from the group consisting of caspase activation, cell death, or cellular DNA degradation over time.

11. A method of screening a compound for viral reaper modulating activity, comprising:
(a) obtaining a population of cells transfected with an isolated nucleotide molecule encoding a viral reaper protein, where said viral reaper protein has at least 70% sequence similarity to SEQ ID NO:2, and is capable of inducing caspase activation in a vertebrate cell;
(b) administering to a test population of said cells a test compound; and
(c) comparing apoptosis occurring in said test population of cells to that which would be expected in a population of cells that did not receive said test compound;
where a reduction in apoptosis indicates that said test compound inhibits viral reaper-induced apoptosis, and an increase in apoptosis indicates said test compound enhances viral-reaper induced apoptosis.

12. A method according to claim 11 where said population of cells consists of insect cells.

13. A method according to claim 11 where said population of cells consists of vertebrate cells.

14. A method according to claim 11 where apoptosis is measured by a criterion selected from caspase activation, cell death, or cellular DNA degradation over time.

15. A method according to claim 11 where said isolated viral reaper protein comprises an amino acid sequence selected from SEQ ID NOs: 2-17.

16. A method according to claim 11 where said isolated viral reaper protein is a non-structural protein (NSs) encoded by the viral small RNA from a virus of the Family Bunyaviridae.

17. A method of inducing apoptosis in a vertebrate cell in vitro by administering an isolated viral reaper protein to said cell, in an amount sufficient to enhance apoptosis over that which would be seen in the absence of viral reaper protein, where said viral reaper protein has at least 70% sequence similarity to SEQ ID NO:2, and is capable of inducing caspase activation in a vertebrate cell.

18. A method according to claim 17 wherein said vertebrate cell is a mammalian cell.

19. A method of inducing apoptosis in a vertebrate cell in vitro by transfecting said cell with an isolated nucleic acid molecule encoding a viral reaper protein, in an amount sufficient to enhance apoptosis over that which would be seen in the absence of transfection, where said encoded viral reaper protein has at least 70% sequence similarity to SEQ ID NO:2 and is capable of inducing caspase activation in a vertebrate cell.

20. A method according to claim 19 where an expression vector containing an isolated nucleotide sequence encoding a viral reaper protein is administered to said cell.

21. A method according to claim 19, where said isolated viral reaper protein is a non-structural protein (NSs) encoded by the viral small RNA from a virus of the Family Bunyaviridae.

## Patentansprüche

1. Verfahren zur Durchmusterung einer Verbindung zur Bestimmung, ob die Verbindung die durch ein virales Reaper-Protein induzierte Caspase-Aktivierung beeinflußt, umfassend:
a) Erhalten eines Wirbeltier-Zellextrakts, in dem die Zugabe eines isolierten viralen Reaper-Proteins eine nachweisbare Caspase-Aktivierung induziert;
b) Zugeben eines isolierten viralen Reaper-Proteins mit wenigstens 70 % Sequenzähnlichkeit mit SEQ ID NO:2, das Caspase-Aktivierung in einer Wirbeltierzelle induzieren kann, und einer Testverbindung zum Wirbeltier-Zellextrakt;
c) Messung der Caspase-Aktivierung; und
d) Vergleichen der Caspase-Aktivierung, die in Gegenwart der Testverbindung auftritt, mit derjenigen, die in Abwesenheit der Testverbindung erwartet würde;
worin eine Abnahme der Caspase-Aktivierung anzeigt, daß die Verbindung die virale Reaper-induzierte Caspase-Aktivierung hemmt, und eine Zunahme der Caspase-Aktivierung anzeigt, daß die Testverbindung die virale Reaper-induzierte Caspase-Aktivierung steigert.

2. Verfahren gemäß Anspruch 1, worin das isolierte virale Reaper-Protein eine aus SEQ ID NOs:2-17 ausgewählte Aminosäuresequenz umfaßt.

3. Verfahren gemäß Anspruch 1, worin das isolierte virale Reaper-Protein ein Nicht-Strukturprotein (NSs) ist, das durch die virale kleine RNA aus einem Virus der Familie Bunyaviridae codiert wird.

4. Verfahren gemäß Anspruch 1, worin der Wirbeltier-Zellextrakt aus Xenopus oocytes erhalten wird.

5. Verfahren zur Durchmusterung einer Verbindung zur Bestimmung, ob die Verbindung die durch ein virales Reaper-Protein induzierte Apoptose beeinflußt, umfassend:
a) Erhalten einer Population von Zellen, worin die Zellen von einem Typ sind, in dem die Zugabe eines isolierten viralen Reaper-Proteins Apoptose induziert;
b) Verabreichen eines isolierten viralen Reaper-Proteins mit wenigstens 70 % Sequenzähnlichkeit mit SEQ ID NQ:2, das Caspase-Aktivierung in einer Wirbeltierzelle induzieren kann, und einer Testverbindung an die Zellen;
c) Messen der Apoptose, die in der Zellpopulation auftritt; und
d) Vergleichen der Apoptose, die in Gegenwart der Testverbindung auftritt,.mit derjenigen, die in Abwesenheit der Testverbindung erwartet würde;
worin eine Abnahme der Apoptose anzeigt, daß die Verbindung die virale Reaper-induzierte Apoptose hemmt, und eine Zunahme der Apoptose anzeigt, daß die Testverbindung die virale Reaper-induzierte Apoptose steigert.

6. Verfahren gemäß Anspruch 5, worin die Population von Zellen aus Wirbeltierzellen besteht.

7. Verfahren gemäß Anspruch 5, worin das isolierte virale Reaper-Protein eine aus SEQ ID NOs:2-17 ausgewählte Aminosäuresequenz umfaßt.

8. Verfahren gemäß Anspruch 5, worin das isolierte virale Reaper-Protein ein Nicht-Strukturprotein (NSs) ist, das durch die virale kleine RNA aus einem Virus der Familie Bunyaviridae codiert wird.

9. Verfahren zur Durchmusterung einer Verbindung zur Bestimmung, ob die Verbindung Apoptose beeinflußt, die durch ein virales Reaper-Protein induziert wird, umfassend:
a) Erhalten einer Population von Zellen, worin die Zellen von einem Typ sind, in dem die Zugabe eines isolierten viralen Reaper-Proteins Apoptose induziert;
b) Transfizieren der Zellen mit einem Nukleotidmolekül, das ein virales Reaper-Protein mit wenigstens 70 % Sequenzähnlichkeit mit SEQ ID NO:2 codiert, das Caspase-Aktivierung in einer Wirbeltierzelle induzieren kann;
c) Verabreichen einer Testverbindung an die Zellen;
d) Messen der Apoptose, die in der Zellpopulation auftritt;
e) Vergleichen der Apoptose, die in Gegenwart der Testverbindung auftritt, mit derjenigen, die in Abwesenheit der Testverbindung erwartet würde;
worin eine Abnahme der Apoptose anzeigt, daß die Verbindung die virale Reaper-induzierte Apoptose hemmt, und eine Zunahme der Apoptose anzeigt, daß die Testverbindung die virale Reaper-induzierte Apoptose steigert.

10. Verfahren gemäß Anspruch 5, worin die Apoptose durch ein-Kriterium gemessen wird, das aus der Gruppe ausgewählt ist, die aus Caspase-Aktivierung, Zelltod oder zellulärem DNA-Abbäu im Zeitablauf besteht.

11. Verfahren zur Durchmusterung einer Verbindung auf virale Reaper-modulierende Aktivität, umfassend:
(a) Erhalten einer Population von Zellen, die mit einem isolierten Nukleotidmolekül transfiziert sind, das ein virales Reaper-Protein codiert, worin das virale Reaper-Protein wenigstens 70 % Sequenzähnlichkeit mit SEQ ID NO:2 aufweist und Caspase-Aktivierung in einer Wirbeltierzelle induzieren kann;
(b) Verabreichen einer Testverbindung an eine Testpopulation der Zellen; und
(c) Vergleichen der Apoptose, die in der Testpopulation von Zellen auftritt, mit derjenigen, die in einer Population von Zellen erwartet würde, die nicht die Testverbindung erhielten;
worin eine Reduktion der Apoptose anzeigt, daß die Testverbindung die virale Reaper-induzierte Apoptose hemmt, und eine Zunahme der Apoptose anzeigt, daß die Testverbindung die virale Reaper-induzierte Apoptose steigert.

12. Verfahren gemäß Anspruch 11, worin die Population von Zellen aus Insektenzellen besteht.

13. Verfahren gemäß Anspruch 11, worin die Population von Zellen aus Wirbeltierzellen besteht.

14. Verfahren gemäß, Anspruch 11, worin die Apoptose durch ein Kriterium gemessen wird, das aus Caspase-Aktivierung, Zelltod oder zellulärem DNA-Abbau im Zeitablauf ausgewählt ist.

15. Verfahren gemäß Anspruch 11, worin das isolierte virale Reaper-Protein eine aus SEQ ID NOs:2-17 ausgewählt Aminosäuresequenz umfaßt.

16. Verfahren gemäß Anspruch 11, worin das isolierte virale Reaper-Protein ein Nicht-Strukturprotein (NSs) ist, das durch die virale kleine RNA aus einem Virus der Familie Bunyaviridae codiert wird.

17. Verfahren zur Induzierung von Apoptose in einer Wirbeltierzelle in vitro durch Verabreichen eines isolierten viralen Reaper-Proteins an die Zelle in einer Menge, die zur Steigerung der Apoptose gegenüber derjenigen ausreichend ist, die in Abwesenheit des viralen Reaper-Proteins beobachtet würde, worin das virale Reaper-Protein wenigstens 70 % Sequenzähnlichkeit mit SEQ ID NO:2 hat und Caspase-Aktivierung in einer Wirbeltierzelle induzieren kann.

18. Verfahren gemäß Anspruch 17, worin die Wirbeltierzelle eine Säugetierzelle ist.

19. Verfahren zur Induzierung von Apoptose in einer Wirbeltierzelle in vitro durch Transfizieren der Zelle mit einem isolierten Nukleinsäuremolekül, das ein virales Reaper-Protein codiert, in einer Menge, die ausreichend zur Steigerung der Apoptose gegenüber derjenigen ist, die in Abwesenheit der Transfektion beobachtet würde, worin das codierte virale Reaper-Protein wenigstens 70 % Säquenzähnlichkeit mit SEQ ID NO:2 hat und Caspase-Aktivierung in einer Wirbeltierzelle induzieren kann.

20. Verfahren gemäß Anspruch 19, worin ein Expressionsvektor, der eine isolierte Nukleotidsequenz enthält, die ein virales Reaper-Protein codiert, an die Zelle verabreicht wird.

21. Verfahren gemäß Anspruch 19, worin das isolierte virale Reaper-Protein ein Nicht-Strukturprotein (NSs) ist, das durch die virale kleine RNA aus einem Virus der Familie Bunyaviridae codiert wird.

## Revendications

1. Procédé de criblage d'un composé pour déterminer si le composé affecte l'activation des caspases induite par une protéine virale reaper, comprenant les étapes consistant à :
a) obtenir un extrait cellulaire de vertébré dans lequel l'adjonction d'une protéine virale reaper isolée induit une activation détectable des caspases ;
b) ajouter une protéine virale reaper isolée ayant au moins 70 % de similarité de séquence avec SEQ ID No 2 et étant capable d'induire l'activation des caspases dans une cellule de vertébré, et un composé test audit extrait cellulaire de vertébré ;
c) mesurer l'activation des caspases ; et
d) comparer l'activation des caspases qui se produit en présence du composé test avec celle qui serait attendue en l'absence du composé test ;
dans lequel une diminution de l'activation des caspases indique que ledit composé inhibe l'activation des caspases induite par reaper viral, et une augmentation de l'activation des caspases indique que ledit composé test améliore l'activation des caspases induite par reaper viral.

2. Procédé selon la revendication 1, dans lequel ladite protéine virale reaper isolée comprend une séquence d'acides aminés choisie parmi les SEQ ID No 2 à 17.

3. Procédé selon la revendication 1, dans lequel ladite protéine virale reaper isolée est une protéine non structurale (NSs) codée par le petit ARN viral issu d'un virus de la famille Bunyaviridae.

4. Procédé selon la revendication 1, dans lequel ledit extrait cellulaire de vertébré est obtenu à partir d'oxocytes de Xenopus.

5. Procédé de criblage d'un composé pour déterminer si le composé affecte l'apoptose induite par une protéine virale reaper, comprenant les étapes consistant à :
a) obtenir une population de cellules, les cellules d'un type dans lequel l'adjonction d'une protéine virale reaper isolée induit l'apoptose ;
b) administrer une protéine virale reaper isolée ayant au moins 70 % de similarité de séquence avec SEQ ID No 2 et étant capable d'induire l'activation des caspases dans une cellule de vertébré, et un composé test auxdites cellules ;
c) mesurer l'apoptose qui se produit dans ladite population de cellules ; et
d) comparer l'apoptose qui se produit en présence du composé test avec celle qui serait attendue en l'absence du composé test ;
dans lequel une diminution de l'apoptose indique que ledit composé inhibe l'apoptose induite par reaper viral, et une augmentation de l'apoptose indique que ledit composé test améliore l'apoptose induite par reaper viral.

6. Procédé selon la revendication 5, dans lequel ladite population de cellules consiste en cellules de vertébrés.

7. Procédé selon la revendication 5, dans lequel ladite protéine virale reaper isolée comprend une séquence d'acides aminés choisie parmi les SEQ ID No 2 à 17.

8. Procédé selon la revendication 5, dans lequel ladite protéine virale reaper isolée est une protéine non structurale (NSs) codée par le petit ARN viral issu d'un virus de la famille Bunyaviridae.

9. Procédé de criblage d'un composé pour déterminer si le composé affecte l'apoptose induite par une protéine virale reaper, comprenant les étapes consistant à :
a) obtenir une population de cellules, les cellules d'un type dans lequel l'adjonction d'une protéine virale reaper isolée induit l'apoptose ;
b) transfecter lesdites cellules avec une molécule nucléotidique codant pour une protéine virale reaper ayant au moins 70 % de similarité de séquence avec SEQ ID No 2 et étant capable d'induire l'activation des caspases dans une cellule de vertébré ;
c) administrer un composé test auxdites cellules ;
d) mesurer l'apoptose qui se produit dans ladite population de cellules ; et
e) comparer l'apoptose qui se produit en présence du composé test avec celle qui serait attendue en l'absence du composé test ;
dans lequel une diminution de l'apoptose indique que ledit composé inhibe l'apoptose induite par reaper viral, et une augmentation de l'apoptose indique que ledit composé test améliore l'apoptose induite par reaper viral.

10. Procédé selon la revendication 5, dans lequel l'apoptose est mesurée selon un critère choisi dans le groupe consistant en l'activation des caspases, la mort cellulaire, ou la dégradation de l'ADN cellulaire avec le temps.

11. Procédé de criblage d'un composé afin d'évaluer son action de modulation de reaper viral, comprenant les étapes consistant à :
a) obtenir une population de cellules transfectées avec une molécule nucléotidique isolée codant pour une protéine virale reaper, où ladite protéine virale reaper a au moins 70 % de similarité de séquence avec SEQ ID No 2, et est capable d'induire l'activation des caspases dans une cellule de vertébré ;
b) administrer à une population test desdites cellules un composé test ; et
c) comparer l'apoptose qui se produit dans ladite population test de cellules avec celle qui serait attendue dans une population de cellules n'ayant pas reçu ledit composé test ;
dans lequel une diminution de l'apoptose indique que ledit composé inhibe l'apoptose induite par reaper viral, et une augmentation de l'apoptose indique que ledit composé test améliore l'apoptose induite par reaper viral.

12. Procédé selon la revendication 11, dans lequel ladite population de cellules consiste en cellules d'insectes.

13. Procédé selon la revendication 11, dans lequel ladite population de cellules consiste en cellules de vertébrés.

14. Procédé selon la revendication 11, dans lequel l'apoptose est mesurée selon un critère choisi parmi l'activation des caspases, la mort cellulaire, ou la dégradation de l'ADN cellulaire avec le temps.

15. Procédé selon la revendication 11, dans lequel ladite protéine virale reaper isolée comprend une séquence d'acides aminés choisie parmi les SEQ ID No 2 à 17.

16. Procédé selon la revendication 11, dans lequel ladite protéine virale reaper isolée est une protéine non structurale (NSs) codée par le petit ARN viral issu d'un virus de la famille Bunyaviridae.

17. Procédé d'induction de l'apoptose dans une cellule de vertébré in vitro par l'administration d'une protéine virale reaper isolée dans ladite cellule, en quantité suffisante pour améliorer l'apoptose par rapport à celle qui serait observée en l'absence de protéine virale reaper, dans lequel ladite protéine virale reaper a au moins 70 % de similarité de séquence avec SEQ ID No 2, et est capable d'induire l'activation des caspases dans une cellule de vertébré.

18. Procédé selon la revendication 17, dans lequel ladite cellule de vertébré est une cellule de mammifère.

19. Procédé d'induction de l'apoptose dans une cellule de vertébré in vitro par la transfection de ladite cellule avec une molécule d'acide nucléique isolée codant pour une protéine virale reaper, en quantité suffisante pour améliorer l'apoptose par rapport à celle qui serait observée en l'absence de transfection, dans lequel ladite protéine virale reaper codée a au moins 70 % de similarité de séquence avec SEQ ID No 2, et est capable d'induire l'activation des caspases dans une cellule de vertébré.

20. Procédé selon la revendication 19, dans lequel un vecteur d'expression contenant une séquence nucléotidique isolée codant pour une protéine virale reaper est administré à ladite cellule.

21. Procédé selon la revendication 19, dans lequel ladite protéine virale reaper isolée est une protéine non structurale (NSs) codée par le petit ARN viral issu d'un virus de la famille Bunyaviridae.


### SEQUENCE LISTING

<110> Smith, Gary K.
<120> Viral Cell Death Protein and Uses Therefore
<130> PU3909
<140>
<141>
<160> 34
<170> PatentIn Ver. 2.1
<210> 1
<211> 65
<212> PRT
<213> Drosophila melanogaster
<400> 1
   Met Ala Val Ala Phe Tyr Ile Pro Asp Gln Ala Thr Leu Leu Arg Glu
    1               5                  10                  15
   Ala Glu Gln Lys Glu Gln Gln Ile Leu Arg Leu Arg Glu Ser Gln Trp
               20                  25                  30
   Arg Phe Leu Ala Thr Val Val Leu Glu Thr Leu Arg Gln Tyr Thr Ser
           35                  40                  45
   Cys His Pro Lys Thr Gly Arg Lys Ser Gly Lys Tyr Arg Lys Pro Ser
       50                  55                  60
   Gln
   65
<210> 2
<211> 92
<212> PRT
<213> Artificial Sequence
<220>
<223> Description of Artificial Sequence: Majority viral reaper sequence
<400> 2
   Met Met Ser His Pro Gln Val Gln Met Asp Leu Ile Leu Met Gln Gly
    1               5                  10                  15
   Met Trp His Leu Val Leu Asn Met Gly Asn Leu Ser Ile Cys Gln Pro
               20                  25                  30
   Leu Gly Ser Ser Ser Leu Met Pro Gln Lys Pro Lys Leu Leu Ser Leu
           35                  40                  45
   Val Ser Arg Arg Gly Lys Leu Ile Leu Asn Leu Glu Ser Gly Arg Trp
       50                  55                  60
   Arg Leu Ser Ile Ile Ile Phe Leu Glu Thr Gly Thr Thr Gln Leu Val
   65                  70                  75                  80
   Thr Thr Ile Leu Pro Ser Thr Gly Phe Gln Asp Ile
   85 90
<210> 3
<211> 97
<212> PRT
<213> San Angelo virus
<400> 3
   Met Met Ser His Gln Pro Val Gln Met Asp Leu Ile Leu Met Gln Gly
   1 5 10 15
   Ile Trp His Ser Val Leu Asn Met Gly Ser Arg Ser Val Cys Leu Gln
   20 25 30
   Leu Gly Ser Ser Ser Ser Met Pro Gln Lys Pro Lys Leu Leu Ser Arg
   35 40 45
   Val Asn Gln Arg Gly Lys Gln Ile Leu Asn Leu Ala Ser Gly Arg Trp
   50 55 60
   Arg Leu Ser Ile Ile Ile Phe Leu Glu Thr Gly Thr Ile Gln Leu Thr
   65 70 75 80
   Thr Ser Ile Leu Pro Ser Thr Asp Cys Leu Asp Thr Trp Leu Asp Gly
   85 90 95
   Phe
<210> 4
<211> 90
<212> PRT
<213> Snowshoe hare virus
<400> 4
   Met Met Ser His Gln Gln Val Gln Met Asp Leu Ile Leu Met Gln Gly
   1 5 10 15
   Ile Trp His Ser Val Leu Asn Met Gln Asn Gln Ser Ile Leu Leu Gln
   20 25 30
   Leu Gly Ser Ser Ser Ser Met Pro Arg Pro Arg Leu Leu Ser Arg Val
   35 40 45
   Ser Gln Arg Gly Arg Gln Ile Leu Asn Leu Glu Ser Gly Arg Trp Arg
   50 55 60
   Leu Ser Ile Ile Ile Phe Leu Glu Thr Gly Thr Ile Gln Leu Thr Thr
   65 70 75 80
   Ile Leu Pro Ser Thr Asp Cys Gln Asp Ile
   85 90
<210> 5
<211> 92
<212> PRT
<213> La Crosse virus
<400> 5
   Met Met Ser His Gln Gln Val Gln Met Asp Leu Ile Leu Met Gln Gly
   1               5                   10                  15
   Ile Trp Thr Ser Val Leu Lys Met Gln Asn His Ser Thr Leu Leu Gln
               20                  25                  30
   Leu Gly Ser Ser Ser Ser Met Leu Gln Arg Pro Arg Leu Leu Ser Arg
           35                  40                  45
   Val Ser Gln Arg Gly Arg Leu Thr Leu Asn Leu Glu Ser Gly Arg Trp
       50                  55                  60
   Arg Leu Ser Ile Ile Ile Phe Leu Glu Thr Gly Thr Thr Gln Leu Val
   65                  70                  75                  80
   Thr Thr Ile Leu Pro Ser Thr Asp Tyr Leu Gly Ile
                   85                  90
<210> 6
<211> 97
<212> PRT
<213> California encephalitis virus
<400> 6
   Met Met Ser His Pro Gln Val Gln Met Asp Leu Ile Leu Met Gln Gly
   1               5                   10                  15
   Met Trp Thr Ser Val Leu Asn Met Gly Asn Gln Leu Thr Leu Leu Gln
               20                  25                  30
   Leu Gly Ser Ser Ser Ser Met Pro Gln Arg Pro Arg Leu Leu Ser Arg
           35                  40                  45
   Val Ser Gln Arg Gly Lys Leu Ile Leu Asn Leu Ala Ser Gly Arg Trp
       50                  55                  60
   Arg Leu Ser Ile Ile Ile Gly Gln Gln Thr Gly Thr Ile Gln Leu Val
   65                  70                  75                  80
   Thr Thr Ile Leu Pro Ser Thr Ala Ser Gln Asp Thr Leu Pro Asp Gly
                   85                  90                  95
   Ser
<210> 7
<211> 92
<212> PRT
<213> Jerry Slough virus
<400> 7
   Met Met Ser His Pro Gln Val Gln Met Asp Leu Ile Gln Met Gln Gly
    1               5                  10                  15
   Leu Trp His Leu Trp Leu Thr Thr Glu Ser Leu Ser Ile Cys Gln Pro
                20                  25                  30
   Leu Gly Ser Ser Ser Leu Met Gln Gln Lys Pro Lys Leu Leu Ser Leu
           35                  40                  45
   Val Asn Arg Ser Gly Lys Leu Leu Leu Ser Leu Glu Ser Gly Arg Trp
       50                  55                  60
   Arg Ser Ser Ile Ile Ile Phe Leu Glu Thr Gly Thr Thr Gln Leu Val
    65                  70                  75                  80
   Thr Thr Ile Leu Pro Ser Ile Gly Phe Gln Asp Ile
                    85                  90
<210> 8
<211> 92
<212> PRT
<213> Jamestown Canyon virus
<400> 8
   Met Met Ser His Pro Gln Val Gln Met Asp Leu Ile Gln Met Gln Gly
    1               5                  10                  15
   Leu Trp His Leu Trp Leu Thr Thr Glu Ser Leu Ser Ile Cys Gln Pro
                20                  25                  30
   Leu Gly Ser Ser Ser Leu Met Gln Gln Lys Pro Lys Leu Leu Ser Leu
           35                  40                  45
   Val Asn Arg Ser Gly Lys Leu Leu Leu Ser Leu Glu Ser Gly Arg Trp
       50                  55                  60
   Arg Ser Ser Ile Ile Ile Phe Leu Glu Thr Gly Thr Thr Gln Leu Val
    65                  70                  75                  80
   Thr Thr Ile Leu Pro Ser Ile Gly Phe Gln Asp Ile
                    85                  90
<210> 9
<211> 92
<212> PRT
<213> Keystone virus
<400> 9
   Met Met Ser His Pro Gln Val Gln Met Asp Leu Ile Leu Met Gln Gly
   1 5 10 15
   Met Trp His Leu Trp Leu Thr Met Gly Ser Arg Ser Val Cys Gln Pro
   20 25 30
   Leu Gly Ser Ser Ser Leu Met Pro Gln Lys Pro Lys Leu Leu Ser Leu
   35 40 45
   Val Ser Arg Ser Gly Arg Leu His Leu Ser Leu Glu Ser Gly Arg Trp
   50 55 60
   Arg Ser Ser Ile Ile Ile Phe Leu Glu Thr Gly Thr Thr Gln Leu Val
   65 70 75 80
   Thr Thr Ile Leu Pro Cys Thr Gly Phe Gln Asp Ile
   85 90
<210> 10
<211> 97
<212> PRT
<213> Melao virus
<400> 10
   Met Met Ser His Gln Gln Val Gln Met Asp Leu Ile Gln Met Gln Gly
   1 5 10 15
   Ile Trp His Leu Gln Leu Arg Met Gly Lys Leu Ser Ile Cys Gln Pro
   20 25 30
   Leu Gly Ser Ser Ser Leu Met Pro Gln Lys Pro Lys Leu Leu Ser Leu
   35 40 45
   Val Asn Arg Arg Gly Lys Leu Leu Leu Asn Leu Glu Thr Gly Arg Trp
   50 55 60
   Lys Leu Ser Thr Ile Ile Phe Leu Glu Thr Gly Thr Thr Gln Leu Val
   65 70 75 80
   Thr Thr Ile Leu Pro Ser Ile Gly Phe Gln Asp Ile Leu Pro Asp Gly
   85 90 95
   Cys
<210> 11
<211> 97
<212> PRT
<213> Trivittatus virus
<400> 11
   Met Met Leu His Gln Gln Val Gln Thr Asp Leu Ile Pro Met Gln Gly
   1 5 10 15
   Met Trp His Leu Leu Leu His Met Pro Asp Arg Thr Ile Phe Leu Leu
   20 25 30
   Leu Gly Ser Ser Ser Ser Met Leu Pro Arg Pro Arg Met Leu Ser Arg
   35 40 45
   Glu Asn Gln Arg Gly Arg Leu Val Leu Asn Leu Ala Ser Gly Arg Trp
   50 55 60
   Arg Trp Ser Ile Ile Ile Phe Leu Ala Thr Gly Thr Ile Gln Leu Val
   65 70 75 80
   Thr Thr Ile Leu Pro Ser Thr Glu Phe Gln Ala Ile Ser Gln Asp Gly
   85 90 95
   Phe
<210> 12
<211> 97
<212> PRT
<213> Morro Bay virus
<400> 12
   Met Met Ser His Pro Gln Val Gln Met Asp Leu Ile Leu Met Gln Gly
   1 5 10 15
   Met Trp Thr Ser Val Leu Asn Met Gly Asn Arg Leu Thr Leu Leu Gln
   20 25 30
   Leu Gly Ser Ser Ser Ser Met Pro Gln Lys Pro Arg Leu Leu Ser Arg
   35 40 45
   Val Ser Gln Arg Gly Lys Leu Ile Leu Asn Leu Ala Ser Gly Arg Trp
   50 55 60
   Arg Leu Ser Ile Ile Ile Phe Gln Gln Thr Gly Thr Ile Gln Leu Val
   65 70 75 80
   Thr Thr Ile Leu Pro Ser Thr Ala Ser Gln Asp Thr Leu Pro Asp Gly
   85 90 95
   Ser
<210> 13
<211> 92
<212> PRT
<213> Inkoo virus
<400> 13
   Met Met Ser His Pro Gln Val Gln Met Asp Leu Ile Gln Met Gln Gly
    1               5                  10                  15
   Leu Trp His Leu Trp Leu Thr Met Glu Asn Leu Leu Ile Trp Gln Pro
               20                  25                  30
   Leu Gly Ser Ser Ser Leu Met Gln Gln Lys Pro Lys Leu Leu Ser Leu
           35                  40                  45
   Val Asn Arg Ser Gly Lys Leu Leu Leu Asn Leu Glu Ser Gly Arg Trp
       50                  55                  60
   Arg Leu Ser Ile Ile Ile Phe Leu Glu Thr Gly Thr Thr Gln Leu Val
   65                  70                  75                  80
   Thr Thr Ile Leu Pro Ser Thr Gly Phe Leu Asp Thr
                   85                  90
<210> 14
<211> 92
<212> PRT
<213> Serra do Navio virus
<400> 14
   Met Met Ser His Gln Pro Val Gln Met Asp Leu Ile Gln Met Gln Gly
    1               5                  10                  15
   Leu Trp His Leu Trp Leu Val Met Gly Ser Arg Ser Ile Leu Gln Pro
               20                  25                  30
   Leu Glu Ser Ser Ser Leu Met Pro Gln Lys Pro Lys Leu Leu Ser Leu
           35                  40                  45
   Ala Ser Arg Arg Gly Lys Leu Leu Leu Ser Leu Glu Thr Gly Arg Trp
       50                  55                  60
   Arg Leu Ser Ile Ile Ile Phe Leu Glu Thr Gly Thr Thr Gln Leu Val
   65                  70                  75                  80
   Thr Thr Ile Leu Pro Ser Thr Glu Phe Gln Asp Ile
                   85                  90
<210> 15
<211> 92
<212> PRT
<213> South River virus
<400> 15
   Met Met Ser His Pro Gln Val Gln Met Asp Leu Ile Gln Met Gln Gly
   1 5 10 15
   Leu Trp His Leu Trp Leu Thr Met Glu Asn Leu Ser Ile Cys Gln Pro
   20 25 30
   Leu Gly Ser Ser Ser Leu Met Gln Gln Lys Pro Lys Leu Leu Ser Leu
   35 40 45
   Val Asn Arg Ser Gly Arg Leu Ile Leu Asn Leu Glu Ser Gly Arg Trp
   50 55 60
   Arg Leu Ser Ile Ile Ile Phe Leu Glu Thr Gly Thr Thr Gln Leu Val
   65 70 75 80
   Thr Thr Ile Leu Pro Ser Thr Gly Phe Leu Asp Ile
   85 90
<210> 16
<211> 97
<212> PRT
<213> Lumbo virus
<400> 16
   Met Met Ser His Pro Pro Val Gln Met Asp Leu Ile Leu Met Gln Gly
   1 5 10 15
   Met Trp Thr Phe Val Leu Asn Met Glu Asn Gln Ser Ile Ser Ile Pro
   20 25 30
   Leu Gly Ser Phe Ser Leu Met Pro Leu Arg Pro Arg Leu Leu Ser Leu
   35 40 45
   Val Ser Arg Arg Gly Arg Leu Val Leu Asn Leu Glu Ser Gly Arg Trp
   Arg Ser Ser Ile Ile Ile Phe Leu Glu Thr Gly Thr Thr Gln Leu Ile
   65 70 75 80
   Thr Thr Ile Leu Pro Ser Thr Gly Cys Gln Gly Ile Trp Leu Asp Gly
   85 90 95
   Cys
<210> 17
<211> 97
<212> PRT
<213> Tahyna virus
<400> 17
   Met Met Ser His Pro Pro Val Gln Met Asp Leu Ile Leu Met Gln Gly
   1 5 10 15
   Met Trp Thr Ser Val Leu Asn Met Gly Lys Gln Leu Ile Ser Ile Pro
   20 25 30
   Leu Gly Ser Ser Ser Leu Met Pro Gln Lys Pro Lys Leu Leu Ser Leu
   35 40 45
   Val Ser Arg Arg Gly Arg Leu Val Leu Asn Leu Glu Ser Gly Arg Trp
   50 55 60
   Arg Ser Ser Ile Ile Ile Phe Leu Glu Thr Gly Thr Thr Gln Leu Ile
   65 70 75 80
   Thr Thr Ile Leu Pro Ser Thr Gly Cys Thr Gly Ile Trp Leu Asp Gly
   85 90 95
   Cys
<210> 18
<211> 23
<212> PRT
<213> Artificial Sequence
<220>
<223> Description of Artificial Sequence: portion of Drosophila melanogaster Grim protein
<400> 18
   Met Ala Ile Ala Phe Tyr Ile Pro Asp Gln Ala Gln Leu Leu Ala Arg
   1 5 10 15
   Ser Tyr Gln Gln Asn Gly Gln
   20
<210> 19
<211> 198
<212> DNA
<213> Drosophila melanogaster
<400> 19
   atggcagtgg cattctacat acccgatcag gcgactctgt tgcgggaggc ggagcagaag 60
   gagcagcaga ttctccgctt gcgggagtca cagtggagat tcctggccac cgtcgtcctg 120
   gaaaccctgc gccagtacac ttcatgtcat ccgaagaccg gaagaaagtc cggcaaatat 180
   gcgaagccat cgcaatga 198
<210> 20
<211> 294
<212> DNA
<213> San Angelo virus
<400> 20
   atgatgtcgc atcaaccggt gcaaatggat ttgatcctga tgcagggtat ctggcattct 60
   gtgttaaaca tggggagtcg atcagtttgt cttcagttag gatcttcttc ctcaatgccg 120
   caaaagccaa agctgctctc tcgcgtaaac cagagaggaa agcaaatcct aaatttggcg 180
   agtggcaggt ggagattgtc aataatcatt ttcctggaaa caggaacaat ccaattgaca 240
   acctcgatct taccatccac agattgtctg gatacctggc tagatgggtt ctag       294
<210> 21
<211> 279
<212> DNA
<213> Snowshoe hare virus
<400> 21
   atgatgtcgc atcaacaggt gcaaatggat ttgatcctga tgcagggtat atggcattct 60
   gtgttaaata tgcagaatca gtcaatcttg ctgcagttag gatcttcttc ctcaatgccg 120
   caaaggccaa ggctgctctc tcgcgtaagc cagagaggaa ggcaaatcct aaatttggag 180
   agtggcaggt ggaggttgtc aataatcatt ttcctggaaa caggaacaat ccaattaaca 240
   gcgacgatct taccatccac agattgtcag gatatttag                        279
<210> 22
<211> 279
<212> DNA
<213> LaCrosse Virus
<400> 22
   atgatgtcgc atcaacaggt gcaaatggat ttgatcctga tgcagggtat atggacttct 60
   gtgttaaaaa tgcagaatca ctcaaccttg ctgcagttag gatcttcttc ctcaatgctg 120
   caaaggccaa ggctgctctc tcgcgtaagc cagagaggaa ggctaaccct aaatttggag 180
   agtggcaggt ggaggttatc aataatcatt ttcctggaaa caggaacaac ccaattggta 240
   acaacgatct taccatccac agattatctg ggtatttag                        279
<210> 23
<211> 294
<212> DNA
<213> California encephalitis virus
<400> 23
   atgatgtcgc atccacaggt gcaaatggat ttgatcctga tgcagggtat gtggacttct 60
   gtgctaaaca tggggaatca attaaccttg ctgcagttag gatcttcttc ctcaatgccg 120
   caaaggccaa ggctgctctc tcgcgtaagc cagagaggaa agctaatcct aaatttggcg 180
   agtggcaggt ggaggttgtc aataatcatt ttccagcaaa caggaacaat ccaattggta 240
   acaacgatct taccatccac cgcatctcag gataccttgc cagatgggtc ctag       294
<210> 24
<211> 279
<212> DNA
<213> Jerry Slough virus
<400> 24
   atgatgtcgc atccacaggt gcaaatggat ttgatccaga tgcagggttt gtggcattta 60
   tggctgacca cggagagtct atcaatctgt cagccgttag gatcttcttc cttaatgcag 120
   caaaagccaa agctgctctc gctcgtaaac cggagcggaa agctactcct aagtttggag 180
   agtggcaggt ggagatcatc aataatcatt ttcctggaaa caggaacaac ccaattggta 240
   acaacgatct taccatccat aggctttcag gatatctag 279
<210> 25
<211> 279
<212> DNA
<213> Jamestown Canyon virus
<400> 25
   atgatgtcgc atccacaggt gcaaatggat ttgatccaga tgcagggttt gtggcattta 60
   tggctgacca cggagagtct atcaatctgt cagccgttag gatcttcttc cttaatgcag 120
   caaaagccaa agctgctctc gctcgtaaac cggagcggaa agctactcct aagtttggag 180
   agtggcaggt ggagatcgtc aataatcatt tttctggaaa caggaacaac ccaattggta 240
   acaacgatct taccatccat aggctttcag gatatctag 279
<210> 26
<211> 279
<212> DNA
<213> Keystone virus
<400> 26
   atgatgtcgc atccacaggt gcaaatggat ttgatcctga tgcagggtat gtggcattta 60
   tggctaacca tggggagtcg atcagtctgt caaccgttag gatcttcttc cttaatgccg 120
   caaaagccaa agctgctctc actcgtaagc cggagcggaa ggctacacct aagtttggag 180
   agtggcaggt ggagatcgtc aataatcatt ttcctggaaa caggaacaac ccaattggta 240
   acaacgatct taccttgcac cggatttcag gatatctag 279
<210> 27
<211> 294
<212> DNA
<213> Melao virus
<400> 27
   atgatgtcgc atcaacaggt gcaaatggat ttgatccaga tgcagggtat ctggcattta 60
   caattgcgca tggggaagct atcaatttgt cagccgttag gatcttcttc cttaatgccg 120
   caaaagccaa agctgctctc tctcgtaaac cggagaggaa agctactcct aaatttggag 180
   actggcaggt ggaaattgtc aacaatcatt ttcctggaaa caggaacaac ccaattggta 240
   acaacgatct taccatccat cggctttcag gatatcttgc cagatgggtg ctag 294
<210> 28
<211> 294
<212> DNA
<213> Trivittatus virus
<400> 28
   atgatgctcc atcaacaggt gcaaacggat ttgatcccga tgcagggtat gtggcattta 60
   ttgctgcaca tgccggatcg tacgatcttt ctgctgttag gatcttcttc ctcaatgctg 120
   ccaaggccaa gaatgctctc tcgagaaaac cagaggggaa ggttagtatt aaatttggcg 180
   agtggtcggt ggaggtggtc aataatcatt ttcctggcaa caggaacaat ccaattggta 240
   acaacgatct taccatccac agaatttcag gctatctcgc aagatgggtt ctag       294
<210> 29
<211> 294
<212> DNA
<213> Morro Bay virus
<400> 29
   atgatgtcgc atccacaggt gcaaatggat ttgatcctga tgcagggtat gtggacttct 60
   gtgctaaaca tggggaatcg attaaccttg ctgcagttag gatcttcttc ctcaatgccg 120
   caaaagccaa ggctgctctc tcgcgtaagc cagagaggaa agctaatcct aaatttggcg 180
   agtggcaggt ggagattgtc aataatcatt ttccagcaaa caggaacaat ccaattggta 240
   acaacgatct taccatccac cgcatctcag gataccttgc cagatgggtc ctag       294
<210> 30
<211> 279
<212> DNA
<213> Inkoo virus
<400> 30
   atgatgtcgc atccacaggt gcaaatggat ttgatccaga tgcagggttt gtggcattta 60
   tggctgacca tggagaatct attaatttgg cagccgttag gatcttcttc cttaatgcag 120
   caaaagccaa agctgctctc gctcgtaaac cggagcggaa agctactcct aaatttggag 180
   agtggcaggt ggagattgtc aataatcatt ttcctggaaa caggaacaac ccaattggta 240
   acaacgatct taccatccac cggctttctg gatacttag                        279
<210> 31
<211> 279
<212> DNA
<213> Serra do Navio virus
<400> 31
   atgatgtcgc atcaaccggt gcaaatggat ttgatccaga tgcagggttt gtggcattta 60
   tggctggtca tggggagtcg atcaatctta cagccgttag aatcttcttc cttaatgccg 120
   caaaagccaa agctgctctc tctcgcaagc cggagaggaa agctactcct aagtttggag 180
   actggcaggt ggagattgtc aataatcatt ttcctggaaa caggaacaac ccaattggta 240
   acaacgatct taccatccac agaatttcag gatatttag                        279
<210> 32
<211> 279
<212> DNA
<213> South River virus
<400> 32
   atgatgtcgc atccacaggt gcaaatggat ttgatccaga tgcagggttt gtggcattta 60
   tggctgacca tggagaatct atcaatctgt cagccgttag gatcttcttc cttaatgcag 120
   caaaagccaa agctgctctc gctcgtaaac cggagcggaa ggctaatcct aaatttggag 180
   agtggcaggt ggagattgtc aataatcatt ttcctggaaa caggaacaac ccaattggta 240
   acaacgatct taccatccac cggctttctg gatatttag                        279
<210> 33
<211> 294
<212> DNA
<213> Lumbo virus
<400> 33
   atgatgtcgc atccaccggt gcaaatggat ttgatcctga tgcagggtat gtggactttt 60
   gtgttaaaca tggagaatca atcaatctcc attccgttag gatctttttc cttaatgccg 120
   ctaaggccaa ggctgctctc gctcgtaagc cggagaggaa ggctagtcct aaatttggag 180
   agtggcaggt ggagatcgtc aataatcatt ttcctggaaa caggaacaac ccaattgata 240
   acaacgatct taccatccac cggctgtcag ggtatctggc tagatgggtg ttag       294
<210> 34
<211> 294
<212> DNA
<213> Tahyna virus
<400> 34
   atgatgtcgc atccaccggt gcaaatggat ttgatcctga tgcagggtat gtggacttct 60
   gtattaaaca tggggaagca attaatctcc attccgttag gatcttcttc cttaatgccg 120
   caaaagccaa agctgctctc gctcgtaagc cggagaggaa ggctagtcct aaatttggag 180
   agtggcaggt ggaggtcgtc aattatcatt ttcctggaaa caggaacaac ccaattgata 240
   acaacgatct taccatccac cggctgtacg ggtatttggc tagatgggtg ctag       294